# EUROPEAN PATENT SPECIFICATION

(11) **EP 1 966 326 B1**
(45) Date of publication and mention of the grant of the patent: **23.04.2014**
(21) Application number: 05856287.7
(22) Date of filing: 30.12.2005
(51) Int. Cl.: C09B 69/00

(54) **AMPHIPHILIC SQUARAINE DYES, PROCESS FOR PREPARATION THEREOF AND USE THEREOF**
AMPHIPHILE SQUARAINFARBSTOFFE, IHRE HERSTELLUNG UND IHRE VERWENDUNG
COLORANTS AMPHIPHILES A BASE DE SQUARAINE ET LEURS PROCEDES DE PREPARATION ET D'UTILISATION

(43) Date of publication of application: 10.09.2008
(73) Proprietor: Council of Scientific and Industrial Research, New Delhi 110 011 (IN)
(72) Inventor: DANABOYINA, Ramaiah, Kerala (IN); KALLIAT, Thazhathveetil, Arun, Kerala (IN); JYOTHISH, Kuthanapillil, Kerala (IN)
(74) Representative: Gregory, Robert John H.
(86) International application number: PCT/IN2005/000457
(87) International publication number: WO 2007/077566

(56) References cited:
- WO-A-2005/047225
- GB-A- 2 370 581
- LI, CHAO; WANG, WEIBO; WANG, XUESONG: "Molecular Design of Squaraine Dyes for Efficient Far-red and Near_IR Sensitization of Solar Cells" CHEMISTRY LETTERS, vol. 34, no. 4, 12 March 2005 (2005-03-12), pages 554-555, XP008073001 Japan
- ROS-LIS ET. AL.: "Squaraines as Fluoro-Chromogenic Probes for Thiol-Containing Compounds and Their Application to the Detection of Biorelevant Thiols" JOURNAL OF THE AMERICAN CHEMICAL SOCIETY, vol. 126, no. 13, 10 March 2004 (2004-03-10), pages 4064-4065, XP002412163
- HUIJUAN CHEN, WILLIAM G. HERKSTROETER, JEROME PERLSTEIN, KOCK-YEE LAW, AND DAVID G. WHITTEN: "Aggregation of a Surfactant Squaraine in Langmuir-Blodgett Films, Solids, and Solution" THE JOURNAL OF PHYSICAL CHEMISTRY, vol. 98, no. 19, 1994, pages 5138-5146, XP002412164
- CHEN, H.; FARAHAT, M. S.; LAW, K.-Y.; WHITTEN, D. G.: "Aggregation of Surfactant Squaraine Dyes in Aqueous Solution and Microheterogeneous Media: Correlation of Aggregation Behavior with Molecular Structure" JOURNAL OF THE AMERICAN CHEMICAL SOCIETY, vol. 118, no. 11, 31 December 1996 (1996-12-31), pages 2584-2594, XP002412165

## Description

The present invention relates to amphiphilic squaraine dyes of the general formulae 1 as shown below 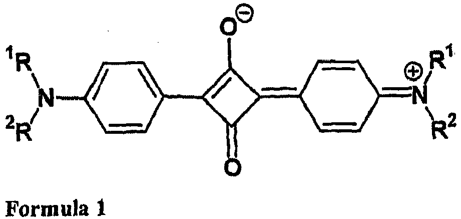 wherein, R¹ = -(CH₂-CH₂-O)ₙ-CH₃, n = 4-8, or -(CH₂)ₙ-CO₂X, n = 3-6, X = H, succinamide and R² = -CH₃ or -(CH₂-CH₂-O)ₙ-CH₃, n = 4-8
and pharmaceutically acceptable derivatives thereof, for use as near infrared fluorescence probes in photodynamic diagnostic, biological and biochemical applications.

The present invention also relates to squaraine dyes of the general formula 1 or pharmaceutically acceptable derivatives thereof, for use as near infrared fluorescence probes in photodynamic applications for the detection of cancer and other diseases in human beings or animals.

The present invention also relates to squaraine dyes of general formula 1 or pharmaceutically acceptable derivatives thereof for use as near-infrared fluorescence probes for biological applications. The present investigation also relates to squaraine dyes of the general formula **1** that can be used as near infrared fluorescent probes for protein labeling. The present investigation also relates to squaraine dyes of the general formula **1** that can be used as near infrared fluorescent labels in immunoassays.

Li et al, Chemistry Letters, Vol 34, No. 4 (2005), 554-555, discloses squaraine dyes for use in the photosensitization of nanocrystalline semiconductor solar cells. One of the compounds disclosed in this document is a compound of formula 1 wherein R¹ is CH₂CH₂CH₂COOH and R² is CH₃.

The present invention further relates to compounds of formula 1 per se, and pharmaceutically derivatives thereof, with the proviso that, when R¹ is CH₂CH₂CH₂COOH, R² is not CH₃.

The present invention further relates to a method of preparing said compounds.

### BACKGROUND OF THE INVENTION

Photodynamic therapy (PDT) is an emerging modality for the diagnosis and treatment of cancer and various diseases, which involves the combined action of light and a photosensitizer. References may be made to Lane, N. Scientific American 2003, 38-45; Bonnett, R. Chem. Soc. Rev. 1995, 24, 19; Dougherty, T. J. Photochem. Photobiol. 1987, 45, 879; Kessel, D.; Dougherty, T. J. Phorphyrin Photosensitization; Plenum Publishing Corp. New York, 1983. The process requires the presence of a photosensitizing agent, which is capable of being taken up by target tissues and which, on irradiation by light of a particular wavelength, generates species which are toxic to those tissues. Photodynamic therapy has advantages over many other conventional therapies due to the selectivity of the photodynamic process. There is more sensitizer in the tumor tissues than in the normal tissues; this reduces the potential for destruction of normal tissues. In addition the ability to direct light specifically onto the target cells and tissues by the use of fiber-optic technology further increased the selectivity of this process. Also, use of photosensitizing agents, which produce no response until irradiated with light, significantly reduces the potential for side effects.

In PDT, the detection of tumor tissue (diagnosis) is equally important when compared to the necrosis of tumor cells (treatment). Near-infrared (NIR) dyes are presently attracting considerable interest as fluorescence probes for the detection of cancer. References may be made to Lin, Y.; Weissleder, R.; and Tung, C. H. Bioconjugate Chem. 2002 13, 605-610; Achilefu, S.; Jimenez, H. N.; Dorshow, R B.; Bugaj, J. E.; Webb, E. G.; Wilhelm, R R; Rajagopalan, R; Johler, J.; Erion, J. L. J. Med. Chem. 2002 45, 2003-2015; Mujumdar, S. R.; Mujumdar, R B.; Grant, C. M.; Waggoner, A. S. Bioconjugate Chem. 1996, 7, 356-362. Since tissue is relatively transparent to NIR light, NIR fluorescence imaging (NIRF) and PDT are capable of detecting and treating, respectively, even subsurface tumors. In this context the present invention aims at the development of efficient near infrared absorbing fluorescent probes based on squaraine dyes for biological applications. We have synthesized dyes based on squaraine moiety which exhibit absorption and emission in the near infrared region and have substituents like carboxyl and glycolic groups, which would render them amphiphilicity thereby increasing their solubility, fluorescence intensity and accelerating their cellular uptake.

In a diagnostic technique, a dye is administered and allowed to distribute in the body as in the case of the treatment technique. However, in addition to the tumor selectivity, the sensitizer in the diagnostic technique should exhibit significant fluorescence yields under physiological conditions. Hence the development of photosensitizers, which have strong absorption in the long wavelength region, nontoxic to normal tissues, soluble in buffer at physiological pH, and exhibit higher therapeutic efficacy are still desired. Also the design of functional molecules that can target specific cancer cells are extremely important because of the biochemical and biomedical applications.

Our interest in this area originated from the idea of utilizing the squaraine dyes for photodynamic applications. Squaraines form a class of dyes possessing sharp and intense absorption bands in the red to near infra red region. The photophysical and photochemical properties of these have been studied extensively, because their absorption and photochemical characteristics make them highly suitable for a number of industrial applications. References may be made to U.S. Pat Nos. 6,001,523; 5,552,253; 5,444,463; Law, K.-Y. Chem. Rev. 1993, 93, 449; Piechowski, A P; Bird, G. R.; Morel, D L.; Stogryn, E. L. J. Phy. Chem. 1984, 88, 934. Preliminary investigations by us indicated that substitution of the squaraine dyes with heavy atoms like bromine and iodine results in their increased solubility in the aqueous medium and enhanced intersystem crossing efficiency, when compared to the parent squaraine dye. These dyes exhibited absorption in the range from 600-620 nm and showed quantum yields of triplet excited states (Φ_{T} = 0.22-0.5) and singlet oxygen (Φ(¹O₂) = 0.13-0.47), depending on the nature of the halogen atoms. The cytotoxicity and mutagenicity studies using mammalian cell lines and bacterial strains indicated that these dyes exhibit significant cytotoxicity upon excitation with visible light and the mechanism of their biological activity could be attributed to the in vitro generation of singlet oxygen. References may be made to Ramaiah, D.; Arun, K. T.; Das, S. and Epe, B. US Patent No. 6,770,787B2 (2004), Ramaiah, D.; Arun, K. T.; Das, S. and Epe, B. Indian patent No. 193540 (2004). Ramaiah, D.; Joy, A.; Chandrasekhar, N; Eldho, N. V.; Das, S.; George, M. V. Photochem. Photobiol. 1997, 65, 783; Arun, K.T.; Ramaiah, D.; Epe, B. J. Phys. Chem. B 2002, 107, 11622, Ramaiah, D.; Eckert, I; Arun, K.T.; Weidenfeller, L.; Epe, B. Photochem. Photobiol. 2002, 76, 672; Ramaiah, D.; Eckert, I; Arun, K.T.; Weidenfeller, L.; Epe, B. Photochem. Photobiol. 2004, 79, 99. However, these heavy atom substituted dyes possesses very low fluorescence quantum yields (Φ_{F} ≤ 0.0003) in aqueous medium, thereby limiting their use for the detection of tumors (diagnosis) by the fluorescence emission of the dyes that can localize selectively in tumor tissues.

In the present invention novel amphiphilic dyes based on squaraine moiety have been synthesized and their potential as near infrared fluorescent probes for biological and biochemical applications has been demonstrated.

### OBJECTIVES OF THE INVENTION

The main objective of the present invention is to provide efficient squaraine based dyes and/or pharmaceutical acceptable derivatives thereof, for use as near infrared sensitizers in photodynamic diagnostic and biochemical applications.

Another objective of the present invention is to provide efficient squaraine based dyes and/or pharmaceutical acceptable derivatives thereof, for use as near infrared fluorescence probes in photodynamic diagnostic applications for the detection of tumors.

Another objective of the present invention is to provide efficient squaraine based dyes and/or pharmaceutical acceptable derivatives thereof, for use as near-infrared fluorescence sensors for biological and biochemical applications.

Yet another objective of the present investigation is to provide squaraine-based dyes that can be used as near infrared fluorescent probes for protein labeling.

Yet another objective of the present investigation is to provide squaraine dyes of the general formula 1 that can be used as near infrared fluorescent labels in immunoassays.

### BRIEF DESCRIPTION OF THE ACCOMPANYING DRAWINGS

In the drawings accompanying the specifications
- **FIG. 1**: Absorption spectra of squaraine dyes of the general formula **1** wherein, R¹ = -(CH₂-CH₂-O)ₙ-CH₃, n = 4 and R² = -CH₃, general formula **2** wherein R¹, R² = -(CH₂-CH₂-O)ₙ-CH₃, n = 4 and general formula **3**, wherein R¹ = -(CH₂)ₙ-CO₂X, n = 3, X = H, and R² = -CH₃, in 10 % vol/vol ethanol water mixtures.
- **FIG. 2**: Fluorescence emission spectra of squaraine dyes of general formula **1**, wherein, R¹ = -(CH₂-CH₂-O)ₙ-CH₃, n = 4 and R² = -CH₃, general formula **2** wherein R¹, R² = - (CH₂-CH₂-O)ₙ-CH₃, n = 4 and general formula **3**, wherein R¹ = -(CH₂)ₙ-CO₂X, n = 3, X = H, and R² = -CH₃, in 10 % vol/vol ethanol water mixtures.
- **FIG. 3**: Emission spectra of squaraine dyes of general formula **1**, wherein R¹ = -(CH₂-CH₂-O)ₙ-CH₃, n = 4 and R² = -CH₃, in the presence of varying concentration of [CTAB] a) 0 and g) 129 mM. Excitation wavelength, 600 nm.
- **FIG. 4**: Emission spectra of squaraine dyes of general formula **1**, wherein R¹ = -(CH₂-CH₂-O)ₙ-CH₃, n = 4 and R² = -CH₃, in the presence of varying concentration of [SDS] a) 0 and e) 21 mM. Excitation wavelength, 600 nm.
- **FIG. 5**: Emission spectra of squaraine dyes of general formula **1**, wherein R¹ = -(CH₂-CH₂-O)ₙ-CH₃, n = 4 and R² = -CH₃, in the presence of varying concentration of [Triton X-100] a) 0 and g) 118 mM. Excitation wavelength, 600 nm.
- **FIG. 6**: Fluorescence decay profile of squaraine dyes of general formula **1** wherein R¹ = - (CH₂-CH₂-O)ₙ-CH₃, n = 4 and R² = -CH₃, in 10% (vol/vol) ethanol-water mixtures. (a) 10% (vol/vol) ethanol-water, (b) triton X-100, (c) CTAB, (d) SDS and (L) lamp profile. Excitation wavelength, 635 nm. Emission wavelength, 670 nm.
- **FIG. 7**: Emission spectra of squaraine dyes of general formula **1**, wherein R¹ = -(CH₂-CH₂-O)ₙ-CH₃, n = 4 and R² = -CH₃, in the presence of varying concentration of [β-CD] a) 0 and g) 25 mM. Excitation wavelength, 600 nm.
- **FIG. 8**: Fluorescence decay profile of squaraine dyes of general formula **1**, wherein R¹ = - (CH₂-CH₂-O)ₙ-CH₃, n = 4 and R² = -CH₃, in 10% (vol/vol) ethanol-water mixtures. [β-CD] (a) 0 (b) 12 mM and (L) lamp profile.

### SUMMARY OF THE INVENTION

Accordingly, the present invention relates to squaraine dyes of the general formula **1** and pharmaceutically acceptable derivatives thereof. 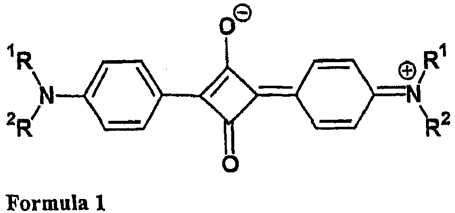
wherein, R¹ = -(CH₂-CH₂-O)ₙ-CH₃, n = 4-8, or -(CH₂)ₙ-CO₂X, n = 3-6, X = H, succinamide
and R² = -CH₃ or -(CH₂-CH₂-O)ₙ-CH₃, n=4-8, for use in various applications, in accordance with the present claims; and furthermore to these compounds per se with the proviso that when R¹ is CH₂CH₂CH₂COOH, R² is not CH₃.

In one embodiment of the invention, the N-methyl-N-substituted or N,N-disubstituted aniline and squaric acid in the ratio 2:1 in a mixture of benzene and n-butanol (1:1) is refluxed at (90-110 °C) for a time period of 18-24 h. Removal of the solvent gave a residue, which was then subjected to column chromatography over silica gel to obtain compounds of the general formula **1**.

Another embodiment of the present invention is to provide efficient squaraine based dyes and/or pharmaceutical acceptable derivatives thereof, for use as near-infrared fluorescence sensors for biological and biochemical applications.

In yet anothe embodiment of the invention, the compounds of the formula 1are used in photodynamic therapy as near infrared fluorescent sensors for the diagnosis of cancer.

Yet another embodiment of the present investigation is to provide squaraine-based dyes that can be used as near infrared fluorescent probes for protein labeling.

Another embodiment of the present investigation is to provide squaraine dyes of the general formula 1 that can be used as near infrared fluorescent labels in immunoassays.

### DETAILED DESCRIPTION OF THE INVENTION

In the present investigation, squaraine dyes of the general formula 1 have been synthesized and their photophysical properties in the presence and absence of membrane mimics like micelles and drug carrier systems like β-cyclodextrin were investigated. In the preparation of the compounds of the general formula 1, the amino protons of the aniline moiety are replaced with methyl, glycol and aliphatic carboxylic acid moieties. Modification with glycol and carboxylic acid moieties is expected to render amphiphilicity to these dyes and hence increase the cell permeability and to bring about target specificity.

The following examples are given by way of illustration and therefore should not be construed to limit the scope of present investigation.

Examples 1-3 represent typical synthesis of compounds of the general formula 1 and examples 4 and 5 represent the photophysical properties of compounds of general formula 1 and in the absence and presence of membrane mimics like neutral, anionic and cationic micelles and drug carriers like β-cyclodextrin.

### EXAMPLE 1

*Preparation of the squaraine dye of general formula **1**, wherein R¹ = -(CH₂-CH₂-O)ₙ-CH₃, n = 4 and R² = -CH₃,* A solution of N-methyl-N-(3,6,9,12-tetraoxatrideca)aniline (400 mg, 1.35 mmol) and squaric acid (77 mg, 0.67 mmol) in a mixture of *n*-butanol and benzene (1:3) was refluxed by azeotropic distillation of water for 18 h. The solvent was distilled off under reduced pressure and the residue obtained was chromatographed over silica gel. Elution of the column with a mixture of methanol and chloroform (1:49) gave 110 mg (15%) of the squaraine dye of the general formula **1**, wherein, R¹ = -(CH₂-CH₂-O)ₙ-CH₃, n = 4 and R² = - CH₃, mp 100-102 °C; ¹H NMR (300 MHz, CDCl₃, 30°C, TMS): δ = 3.21 (s, 6H, -NCH₃), 3.37 (s, 6H, -OCH₃), 3.72-3.52 (m, 32H, -OCH₂), 6.81 (d, 4H, J= 8.96, Ar-H), 8.39 (d, 4H, J = 8.95 Hz, Ar-H); ¹³C NMR (75 MHz, CDCl₃, 30 °C, TMS): δ = 39.68, 52.29, 58.97, 68.53, 70.42, 70.52, 70.56, 70.81, 71.83, 112.43, 119.90, 133.17, 154.41, 183.32, 188.58; IR (Neat): νₘₐₓ 2877, 1610, 1584, 1140, 1098 cm⁻¹; elemental analysis calcd (%) for C₃₆H₅₂N₂O₁₀: C, 64.27; H, 7.79; N, 4.16; found: C, 64.51; H, 7.69; N, 3.88.

### EXAMPLE 2

*Preparation of the squaraine dye of the general formula 2, wherein R¹, R² = -(CH₂-CH₂-O)ₙ*-*CH₃, n* = 4. A solution of bis-(*N*,*N*-(3,6,9,12-tetraoxatrideca)aniline (350 mg, 0.74 mmol) and squaric acid (42 mg, 0.37 mmol) in a mixture of *n*-butanol and benzene (1:3) was refluxed by azeotropic distillation of water for 18 h. The solvent was distilled off under reduced pressure and the residue obtained was chromatographed over silica gel. Elution of the column with a mixture (1:99) of methanol and chloroform gave 40 mg (5%) of the squaraine dye of the general formula 2, wherein R¹, R² = -(CH₂-CH₂-O)ₙ-CH₃, n = 4, mp 78-80 °C; ¹H NMR (300 MHz, CDCl₃, 30 °C, TMS): δ = 3.37 (s, 12H, -OCH₃), 3.77-3.55 (m, 64H, -OCH₂), 6.84 (d, 4H, J = 8.96, Ar-H), 8.37 (d, 4H, J = 8.95 Hz, Ar-H); ¹³C NMR (75 MHz, CDCl₃, 30 °C, TMS): δ = 50.73, 58.91, 68.31, 70.38, 70.49, 70.53, 71.80, 111.54, 115.85, 129.15, 147.60, 183.32, 188.58; IR (Neat): νₘₐₓ 2918, 2867, 1610, 1584, 1114 cm⁻¹; Elemental analysis calcd (%) for C₅₂H₈₄N₂O₁₈: C, 60.92; H, 8.26; N, 2.73; found: C, 61.20; H, 7.98; N, 2.49.

### EXAMPLE 3

*Preparation of squaraine dye of the general formula 3, wherein R¹ = -(CH₂)ₙ-CO₂X, n =* 3, *X = H, and R²* = -*CH₃*. *N*-methyl-*N*-(carboxypropyl) aniline (319 mg, 1.74 mmol) and squaric acid (100 mg, 0.87 mmol) were refluxed in a mixture of *n*-butanol and benzene (1:3) by azeotropic distillation of water for 24 h. The solvent was distilled off under reduced pressure to obtain a residue which was chromatographed over silica gel. Elution of the column with a mixture (1:9) of methanol and chloroform gave 100 mg (13%) of the squaraine dye of the general formaula 3, wherein R¹ = -(CH₂)ₙ-CO₂X, n = 3, X = H, and R² = -CH₃, mp 238-240 °C (d); ¹H NMR (300 MHz, [D₆]DMSO, 30 °C, TMS): δ = 1.91 (p, 4H, -CH₂), 2.40 (t, 4H, J = 7.2 Hz, -CH₂), 2.91(s, 6H, -NCH₃), 3.35 (t, 4H, J = 7.3 Hz, -CH₂), 6.99 (d, 4H, J = 9.07 Hz, Ar-H), 8.05 (d, 4H, J = 8.97 Hz, Ar-H); ¹³C NMR (75 MHz, [D₆]DMSO, 30 °C, TMS): δ = 21.82, 31.41, 38.46, 52.03, 112.64, 116.73, 129.17, 149.13, 179.23; IR (KBr): νₘₐₓ 3420, 2924, 1729, 1590, 1439, 1130 cm⁻¹; elemental analysis calcd (%) for C₂₆H₂₈N₂O₆: C, 67.23; H, 6.08; N, 6.03; found: C, 67.50; H, 5.81; N, 5.80.

### EXAMPLE 4

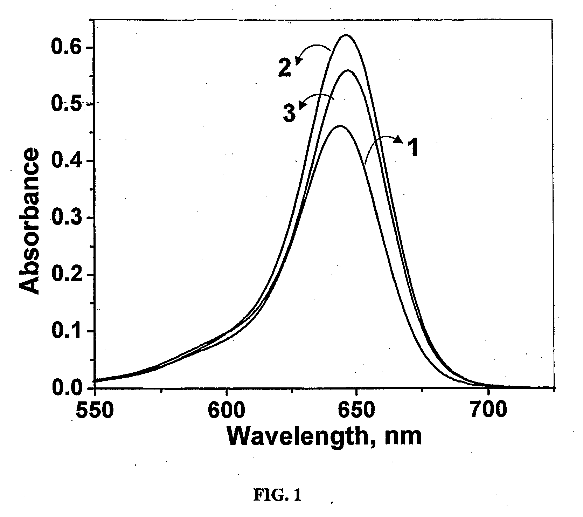

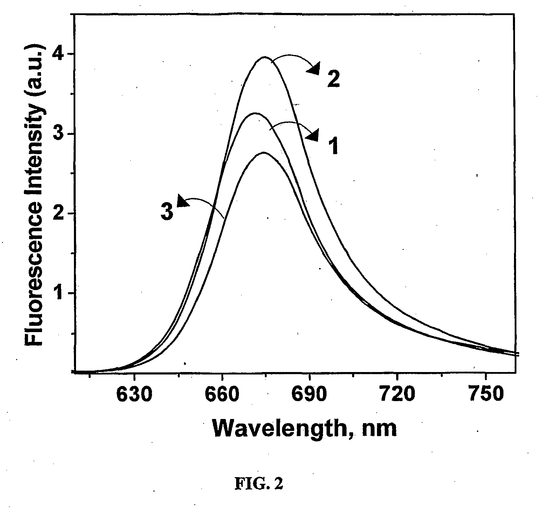

Enormous interest has been generated in the development of water soluble near infrared probes which have absorption in the longer wavelength region where biological chromophores do not absorb. FIG 1. shows the absorption spectra of dyes of the general formula 1 in 10% vol/vol ethanol-water mixtures. As seen from the figures, these dyes showed sharp absorption with absorption maxima around 640-645 nm in aqueous solutions, whereas they showed a bathochromic shift in alcoholic solvents like ethanol with maxima around 640-645. FIG. 2 shows the emission spectra of these dyes in 10% vol/vol ethanol-water mixtures. These dyes exhibited fluorescence emission maxima in the region 670-676 nm in aqueous solutions while the emission maximum was found to be in the region 660-665 nm in alcoholic solvents. These dyes exhibited fluorescence quantum yield in the range 0.007 to. 0.021 in aqueous media while they exhibited fluorescence quantum yield in the range 0.18 to 0.21 in ethanol. The absorption and emission maxima, and fluorescence quantum yields of these dyes in aqueous and alcoholic solvents are listed in Table 1. The long wavelength absorption and emission maxima and good fluorescence quantum yields in aqueous media make these dyes ideal candidates for application as fluorescence probes.

**TABLE 1**

| **Formula** | **Ethanol λₘₐₓ, nm** | | | **10% v/v Ethanol/water λₘₐₓ, nm** | | | **Water λₘₐₓ, nm** | | |
|---|---|---|---|---|---|---|---|---|---|
| | **abs** | **ems** | **Φ_{f}** | **abs** | **ems** | **Φ_{f}** | **abs** | **ems** | **Φ_{f}** |
| **1** | 637 | 660 | 0.19 | 645 | 674 | 0.018 | 644 | 671 | 0.013 |
| **2** | 638 | 662 | 0.21 | 646 | 675 | 0.026 | 646 | 676 | 0.021 |
| **3** | 636 | 662 | 0.18 | 647 | 673 | 0.015 | 645 | 673 | 0.007 |

### EXAMPLE 5

Study of the stability and photophysical properties of a photosensitizer under physiological conditions is important during its evaluation for various *in vitro* and *in vivo* applications. Particularly, the influence of membrane mimics like cetyltrimethylammonium bromide and carrier systems like β-cyclodextrin will provide information on the sensitizer behaviour under physiological conditions. Moreover, such studies are also useful to clarify points such as whether a particular dye forms aggregates or not and whether it generates cytotoxic agents or not in such environments. These media are unique in their properties, since β-CD forms inclusion complexes with the guest molecules, while others form micellar structures thereby provide in them both the hydrophobic and hydrophilic environment.

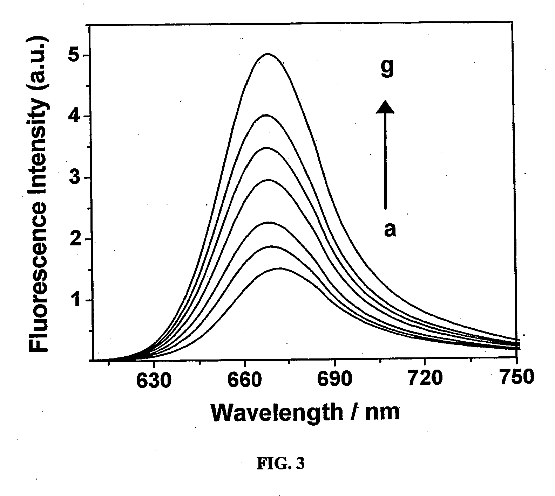

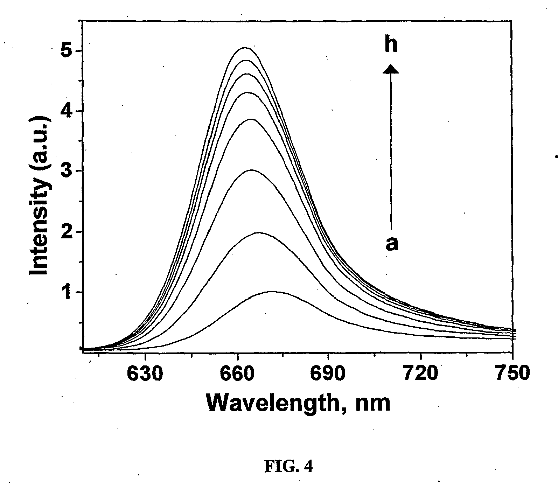

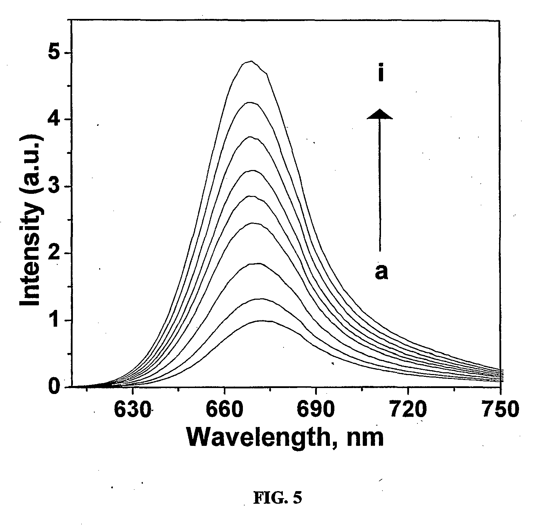

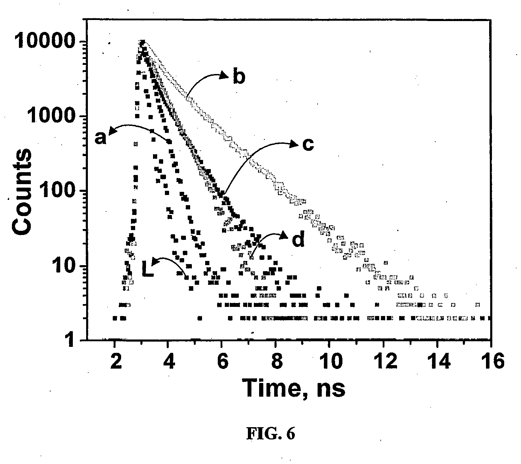

FIGS. 3, 4, and 5 show the emission spectra of dye of the general formula 1, wherein, R¹ = -(CH₂-CH₂-O)ₙ-CH₃, n = 4 and R² = -CH₃ in the presence of cationic micelle CTAB, anionic micelle SDS and neutral micelle Triton X-100, respectively. As the concentration of micelles increases, the fluorescence intensity of the dye increases showing an effective interaction between the micelles and the dye. To have a better understanding of the effect of micellar media we have analyzed the picosecond time-resolved fluorescence lifetimes of dyes of the general formula 1. These dyes showed a mono exponential decay in the absence of micellar media but biexponential decay in the presence of micelles. This biexponential decay in the presence of micelles indicates the existence of two spectroscopically distinct species, one arising from the encapsulated dye molecules and the other arising from unbound dye molecules. FIG 6 shows the fluorescence decay profiles of squaraine dye of the general formula **1** wherein, R¹ = -(CH₂-CH₂-O)ₙ-CH₃, n = 4 and R² = -CH₃ in 10 % vol/vol ethanol-water mixtures and in the presence of cationic micelle CTAB, anionic micelle SDS and neutral micelle Triton X-100. Tables 2, 3 and 4

**TABLE 2**

| **Formula** | **In the presence of CTAB** | | | |
|---|---|---|---|---|
| | **Abs λₘₐₓ, nm** | **Ems λₘₐₓ, nm** | **Φ_{f}** | **Lifetimes, ps** |
| **1** | 642 | 663 | 0.12 | 560 (45%) |
| | | | | 920 (55%) |
| **2** | 644 | 667 | 0.096 | 410 (25%) |
| | | | | 1140 (75%) |
| **3** | 644 | 667 | 0.14 | 440 (4%) |
| | | | | 860 (96%) |

lists the absorption and emission maxima, fluorescence quantum yield and lifetimes of dyes of the general formula **1** wherein, R¹ = -(CH₂-CH₂-O)ₙ CH₃, n = 4 and R² = -CH₃, general formula **2**, wherein R¹, R² = -(CH₂-CH₂-O)ₙ-CH₃, n = 4 and general formula 3, wherein R¹ = -(CH₂)ₙ-CO₂X, n = 3, X = H, and R² = -CH₃, in the absence and presence of cationic micelle CTAB, anionic micelle SDS and neutral micelle Triton X-100. These results demonstrate that these dyes are ideal candidates as near infrared sensors in biological applications where they would effectively interact with cell membrane structures.

**TABLE 3**

| **Formula** | **In the presence of SDS** | | | |
|---|---|---|---|---|
| | **Abs λₘₐₓ, nm** | **Ems λₘₐₓ, nm** | **Φ_{f}** | **Lifetimes, ps** |
| **1** | 640 | 663 | 0.15 | 590 |
| **2** | 643 | 668 | 0.16 | 690 (4%) |
| | | | | 1240 (96%) |

**TABLE 4**

| **Formula** | **In the presence of Triton X-100** | | | |
|---|---|---|---|---|
| | **Abs λₘₐₓ, nm** | **Ems λₘₐₓ, nm** | **Φ_{f}** | **Lifetimes, ps** |
| **1** | 645 | 669 | 0.12 | 130(21%) |
| | | | | 1120 (90%) |
| **2** | 646 | 671 | 0.14 | 450 (7%) |
| | | | | 1450 |
| **3** | 649 | 672 | 0.12 | 430 (31%) |
| | | | | 1140 (69%) |

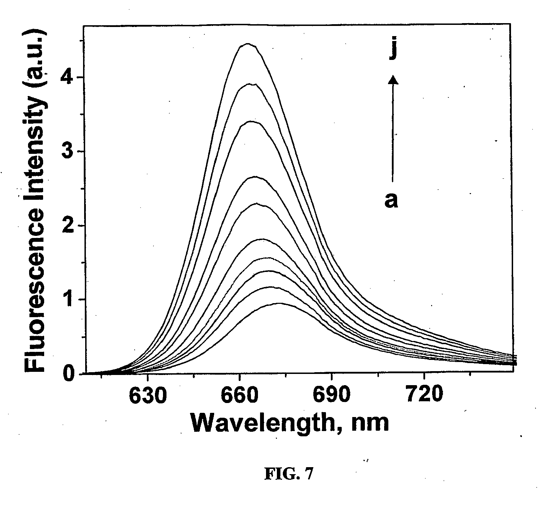

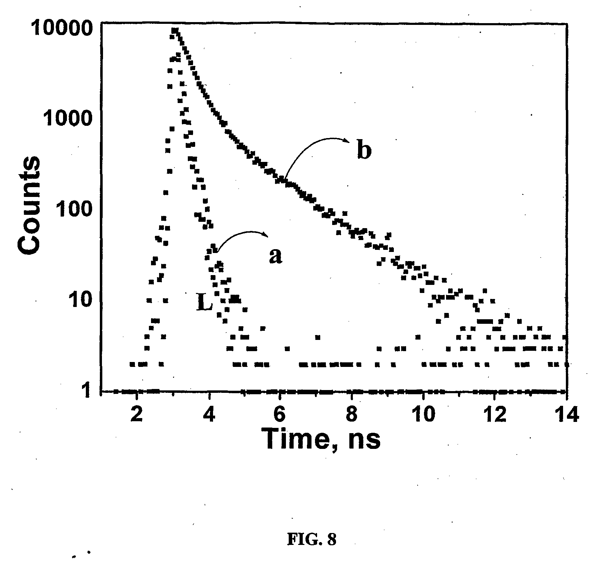

FIG. 7 shows the emission spectra of dye of the general formula 1, wherein, R¹ = - (CH₂-CH₂-O)ₙ-CH₃, n = 4 and R² = -CH₃ in the presence of β-CD. Similar to what was observed with micelles, as the concentration of β-CD increases the emission intensity of the squaraine dyes of general formula 1 increases and the emission maxima shows a blue shift revealing effective interaction between the dyes and β-CD cavity. FIG. 8 shows the fluorescence decay profile of squaraine dye of general formula 1 wherein R¹ = -(CH₂-CH₂-O)ₙ-CH₃, n = 4 and R² = -CH₃ in the absence and presence of β-CD, it showed a mono exponential decay in the absence of β-CD. Biexponential decay was observed in the presence of β-CD indicating the presence of two species, one arising from the dye molecules encapsulated inside the β-CD cavity and the other arising from free dye molecules. Table 5 summarises the absorption and emission maxima, fluorescence quantum yields and lifetimes of squaraine dyes of the general formula 1 in the presence of β-CD. These results clearly indicate that these dyes can be localized on at specific targets using drug delivery systems.

**TABLE 5**

| **Formula** | **In the presence of β-CD** | | | |
|---|---|---|---|---|
| | **Abs λₘₐₓ, nm** | **Ems λₘₐₓ, nm** | **Φ_{f}** | **Lifetime, ps** |
| **1** | 644 | 663 | 0.10 | 380 (58%) |
| | | | | 1470 (42%) |
| **2** | 645 | 668 | 0.07 | 610 (83%) |
| | | | | 1920 (17%) |
| **3** | 649 | 666 | 0.19 | 470 (34%) |
| | | | | 1600 (66%) |

The squaraine dyes of the present invention possess satisfactory properties of a near infrared fluorescent probe in photodynamic, diagnostic, biological and biochemical applications.

The main advantages of these systems include:
1. Squaraines dyes represented by formulae 1 and 2 are novel and pure single substances.
2. Their synthetic mehodology is very economical.
3. Squaraine dyes represented by formulae **1, 2** and **3** possess absorption in the near-infrared region (600-700 nm).
4. Squaraine dyes represented by formulae **1, 2** and **3** possess fluorescence emission in the near-infrared region (620-720 nm).
5. Symmetrical squaraine dyes represented by formulae **1, 2** and **3** possess emission quantum yields in the range 0.015-0.03 in aqueous media and increased nearly 10-fold (Φ_{F} = 0.09-0.2) in the presence membrane mimics and drug carriers.
6. They can be used for photodynamic applications such as sterilization of fluids etc.
7. The squaraine-based dyes can be used as near infrared fluorescent probes for protein labeling.
8. The squaraine dyes of the general formula 1 can be used as near infrared fluorescent labels in immunoassays.
9. They can be used for the detection of biologically important metal ions under physiological conditions.
10. These novel dyes can be used as near-infrared fluorescence sensors in biological and biochemical applications.

## Claims

1. Squaraine dyes of the general formula 1 and/ or pharmaceutically acceptable derivatives thereof 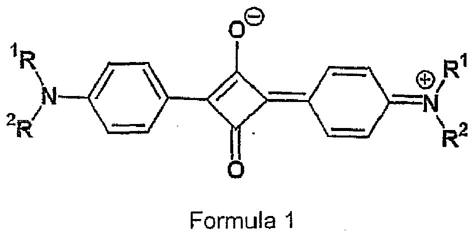 wherein, R¹ = -(CH₂-CH₂-O)ₙ-CH₃, n = 4-8, or -(CH₂)ₙ-CO₂X, n = 3-6, X = H, succinamide and R²= -CH₃ or -(CH₂-CH₂-O)ₙ-CH₃, n = 4-8, with the proviso that, when R¹ = CH₂CH₂CH₂COOH, R² is not CH₃.

2. A process for the preparation Squaraine dyes as claimed in claim 1 and/or pharmaceutically acceptable derivatives thereof 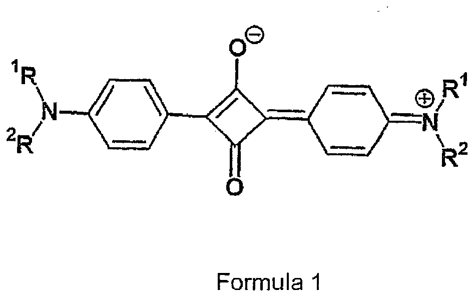 which comprises of reacting N-methyl-N-substituted or N,N-disubstituted aniline with squaric acid in a mixture of benzene and n-butanol and evaporating the solvent and purification of the residue to give compounds of the formula 1.

3. A process as claimed in claim 2 wherein the mixture of benzene and n-butanol is 1:1.

4. A process as claimed in claim 2 or 3 wherein the temperature of the reaction is in the range of 90-110 °C.

5. A process as claimed in claims 2 to 4 wherein the reaction is carried out for a period of 18- 24 h .

6. A process as claimed in claims 2 to 5 wherein the purification is effected by column chromatography over silica gel to obtain compounds of the general formula 1.

7. The compound of formula 1 as claimed in claim 1 without the proviso, for use in near-infrared fluorescence sensors for photodynamic or diagnostic applications.

8. The compound of formula 1 as claimed in claim 1 without the proviso, for use in near-infrared fluorescence probes in photodynamic applications for the detection of cancer and other diseases in human beings or animals.

9. Use of compound as claimed in claim 1 without the proviso, for near-infrared fluorescence probes for biological applications such as protein labeling.

10. Use of compound as claimed in claim 1 without the proviso, for infrared fluorescent labels in immunoassays.

11. Use of compound as claimed in claim 1 without the proviso, for near-infrared fluorescence sensors for biological or biochemical applications.

## Patentansprüche

1. Squarain-Farbstoffe der allgemeinen Formel 1 und/oder pharmazeutisch verträgliche Derivate davon: 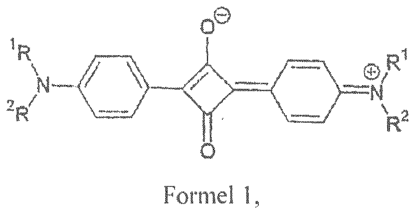 worin R¹ = -(CH₂-CH₂-O)ₙ-CH₃, n = 4-8, oder -(CH₂)ₙ-CO₂X, n = 3-6, X = H, Succinamid und R² = -CH₃ oder -(CH₂-CH₂-O)ₙ-CH₃, n = 4-8, mit der Maßgabe, dass, wenn R¹ = CH₂CHCH₂COOH, R² nicht CH₃ ist.

2. Verfahren zur Herstellung von Squarain-Farbstoffen nach Anspruch 1 und/oder pharmazeutisch verträglichen Derivaten davon: 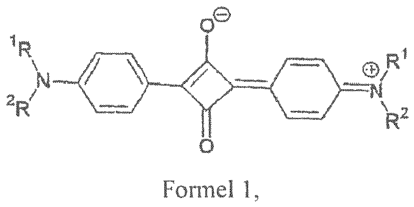 das die Reaktion von N-Methyl-N-substituiertem oder N,N-disubstituiertem Anilin mit Quadratsäure in einer Mischung aus Benzol und n-Butanol und die Verdampfung des Lösungsmittels und die Aufreinigung des Rückstands umfasst, um die Verbindungen der Formel 1 zu erhalten.

3. Verfahren nach Anspruch 2, worin die Mischung aus Benzol und n-Butanol 1 : 1 beträgt.

4. Verfahren nach Anspruch 2 oder 3, worin die Temperatur der Reaktion im Bereich von 90-110°C liegt.

5. Verfahren nach den Ansprüchen 2 bis 4, worin die Reaktion für eine Zeitdauer von 18-24 h durchgeführt wird.

6. Verfahren nach den Ansprüchen 2 bis 5, worin die Aufreinigung durch Säulenchromatographie an Kieselgel ausgeführt wird, um die Verbindungen der allgemeinen Formel 1 zu erhalten.

7. Verbindung der Formel 1 nach Anspruch 1, ohne die Maßgabe, für die Verwendung in Nah-Intrarot-Fluoreszenz-Sensoren für photodynamische oder diagnostische Anwendungen.

8. Verbindung der Formel 1 nach Anspruch 1, ohne die Maßgabe, für die Verwendung in Nah-Infrarot-Fluoreszenz-Sonden in photodynamischen Anwendungen für den Nachweis von Krebs und anderen Erkrankungen bei Menschen oder Tieren.

9. Verwendung der Verbindung nach Anspruch 1, ohne die Maßgabe, für Nah-Infrarot-Fluoreszenz-Sonden für biologische Anwendungen, wie zum Beispiel Proteinmarkierung.

10. Verwendung der Verbindung nach Anspruch 1, ohne die Maßgabe, für Infrarot-Fluoreszenzmarker in Immunoassays.

11. Verwendung der Verbindung nach Anspruch 1, ohne die Maßgabe, für Nah-Infrarot-Fluoreszenz-Sensoren für biologische oder biochemische Anwendungen.

## Revendications

1. Colorants à base de squaraine de formule générale 1, et/ou des dérivés pharmaceutiquement acceptable de ceux-ci 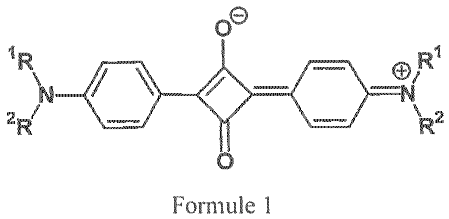 dans laquelle R¹= -(CH₂-CH₂-O)ₙ-CH₃, n= 4-8, ou -(CH₂)ₙ-CO₂X, n= 3-6, X= H, succinamide et R²= -CH₃ ou -(CH₂-CH₂-O)ₙ-CH₃, n= 4-8, à condition que, lorsque R¹= CH₂CH₂CH₂COOH, R² ne soit pas CH₃.

2. Procédé de préparation de colorants à base de squaraine selon la revendication 1, et/ou de dérivés pharmaceutiquement acceptable de ceux-ci 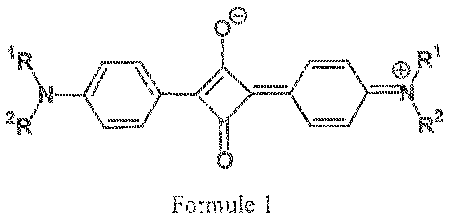 comprenant la réaction d'aniline N-méthyl-N-substituée ou N,N-disubstituée avec de l'acide squarique et dans un mélange de benzène et de n-butanol, et l'évaporation du solvant et la purification du résidu pour donner les composés de formule 1.

3. Procédé selon la revendication 2, dans lequel le mélange de benzène et de n-butanol est de 1:1.

4. Procédé selon la revendication 2 ou 3, dans lequel la température de la réaction se trouve dans la plage de 90-110°C.

5. Procédé selon les revendications 2 à 4, dans lequel la réaction est effectuée pendant une période de 18-24 h.

6. Procédé selon les revendications 2 à 5, dans lequel la purification est effectuée par chromatographie sur colonne sur un gel de silice, afin d'obtenir les composés de formule générale 1.

7. Composé de formule 1 selon la revendication 1, sans la condition, pour une utilisation dans des capteurs de fluorescence proche infrarouge pour des applications photodynamiques ou de diagnostic.

8. Composé de formule 1 selon la revendication 1, sans la condition, pour une utilisation dans des sondes de fluorescence proche infrarouge dans des applications photodynamiques pour la détection de cancer et d'autres maladies chez l'homme ou l'animal.

9. Utilisation du composé selon la revendication 1, sans la condition, pour des sondes de fluorescence proche infrarouge dans des applications biologiques telles que le marquage de protéines.

10. Utilisation du composé selon la revendication 1, sans la condition, pour des marqueurs de fluorescence proche infrarouge dans des immunoessais.

11. Utilisation du composé selon la revendication 1, sans la condition, pour des capteurs de fluorescence proche infrarouge dans des applications biologiques ou biochimiques.
